(19) Europäisches Patentamt
European Patent Office
Office européen des brevets

(11) Veröffentlichungsnummer : **0 148 353**
**B1**

(12) # EUROPÄISCHE PATENTSCHRIFT

(45) Veröffentlichungstag der Patentschrift : **27.05.87**

(51) Int. Cl.⁴ : **C 07 D239/55**

(21) Anmeldenummer : **84113200.4**

(22) Anmeldetag : **02.11.84**

(54) **Verfahren zur Herstellung von Uracil.**

(30) Priorität : **24.11.83 DE 3342419**

(43) Veröffentlichungstag der Anmeldung : **17.07.85 Patentblatt 85/29**

(45) Bekanntmachung des Hinweises auf die Patenterteilung : **27.05.87 Patentblatt 87/22**

(84) Benannte Vertragsstaaten : **AT BE CH DE FR GB IT LI NL SE**

(56) Entgegenhaltungen :
**EP-A- 0 071 018**
**DE-A- 3 247 995**
**US-A- 3 718 649**
**PATENT ABSTRACTS OF JAPAN, Band 5, Nr. 89, 10. Juni 1981 (C-58) (761)**

(73) Patentinhaber : **DYNAMIT NOBEL AKTIENGESELLSCHAFT**
**Postfach 1261**
**D-5210 Troisdorf, Bez. Köln (DE)**

(72) Erfinder : **Peeters, Hermann, Dr.**
**Porzer Strasse 1**
**D-5216 Niederkassel 3 (DE)**
Erfinder : **Vogt, Wilhelm, Dr.**
**Kleiststrasse 39**
**D-5000 Köln 40 (DE)**

**EP 0 148 353 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft die Herstellung von 2,4 (1H, 3H) Pyrimidindion (Uracil) aus Alkaliformylessigsäurealkylestern durch Umsetzung mit Thioharnstoff und anschließender Umsetzung mit Wasserstoffperoxid ohne Isolierung von Zwischenprodukten nach dem Reaktionsschema

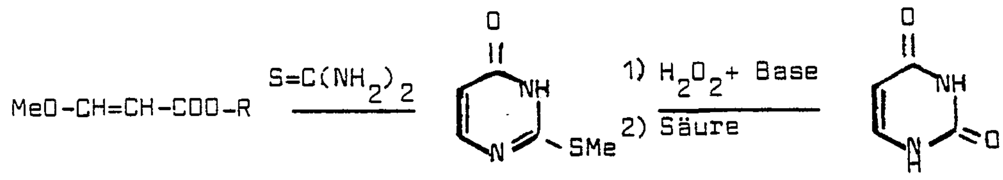


Uracil ist ein Produkt mit breiter Anwendung als Industriechemikalie als Agrochemikalie oder als pharmazeutisches Ausgangsprodukt (Ind. Chem. Prod. Res. Dev. Vol. 17, N° 4 (1978) 278).

Die Herstellung von Uracil ist nach zahlreichen Verfahren möglich, wie z. T. in der oben genannten Literaturstelle angegeben. Die Umsetzung von Äpfelsäure mit Harnstoff in $SO_3$ enthaltender Schwefelsäure hat den Nachteil der Entsorgung bzw. Aufarbeitung großer Mengen an verdünnter organisch belasteter Schwefelsäure. Bei der Verseifung von 2-Thiouracil mit Chloressigsäure und Salzsäure entsteht durch gebildete Mercaptoessigsäure eine starke Geruchsbelästigung und die Gewinnung eines geruchsfreien Uracils ist schwierig und verlustreich.

Es bestand die Aufgabe, Uracil in hoher Ausbeute und Reinheit unter Vermeidung umweltbelastender Abfallstoffe nach einem technisch einfach durchführbaren Verfahren aus einfachen Ausgangsstoffen herzustellen.

Die Aufgabe konnte dadurch gelöst werden, daß Alkaliformylessigsäurealkylester zunächst mit Thioharnstoff und dann mit Wasserstoffperoxid in einer « Eintopfreaktion » bei milden Temperaturen in wäßrigem Medium umgesetzt werden. Überraschend behindern die Nebenprodukte nicht die Reaktion und erfordern keine Isolierung von Zwischenprodukten.

Gegenstand der Erfindung ist ein Verfahren zur Herstellung von Uracil der Formel

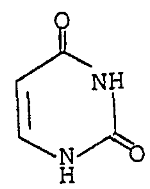

(1)

durch Umsetzung von Alkaliformylessigsäurealkylester

$$MeO—CH=CH—COOR \qquad (2)$$

worin Me ein Alkalimetall, bevorzugt Natrium oder Kalium, und R einen Alkylrest mit 1 bis 8 Kohlenstoffatomen, bevorzugt einen Methyl- oder Ethylrest, bedeuten, mit Thioharnstoff zum Alkalisalz des 2-Mercapto-4-hydroxypyrimidins

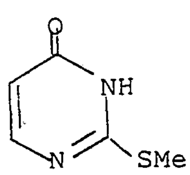

(3)

worin Me dieselbe Bedeutung wie in Formel (2) hat, dadurch gekennzeichnet, daß ohne Isolierung der Stoffe der Formel (3) deren entstandenen Reaktionslösung mit Wasserstoffperoxid in wäßriger Lösung in Gegenwart von mindestens 2 Mol Base zur Reaktion gebracht, anschließend durch Ansäuern auf einen pH-Wert von 6 oder weniger Uracil gebildet und gewonnen wird.

Die Herstellung der Stoffe der Formel (3) ist aus EP-A-0 071 018 bekannt, jedoch erfolgt dort die Umsetzung zu Uracil auf nicht vorteilhafte Weise.

Die Mengen Thioharnstoff betragen 0,95 bis 1,05 Mol je Mol (2). Der Alkaliformylessigsäurealkylester ist durch Formylierung von Essigsäureestern mit Ameisensäureestern oder Kohlenmonoxid zugänglich und kann als Feststoff oder Lösung, sogar zusammen mit Verunreinigungen aus der Herstellung, eingesetzt werden.

Entsprechend dem vorliegenden Verfahren wird Alkaliformylessigsäurealkylester mit Thioharnstoff

zunächst zum Alkalisalz des 4-Hydroxy-2-mercaptopyrimidins, beispielsweise nach der US-PS 3 718 649 und dieses direkt mit Wasserstoffperoxid weiter umgesetzt.

Hierzu kann beispielsweise einer wäßrigen Lösung aus Alkalihydroxid und Thioharnstoff ein Alkaliformylessigsäurealkylester als Feststoff oder in wäßriger Lösung bei Temperaturen von etwa 10 bis 25 °C zudosiert und im Temperaturbereich von 20 bis 100 °C in etwa 1 bis 2 Stunden zur Reaktion gebracht werden. Bei der Reaktion braucht keine freie Base zugesetzt werden, jedoch erhöhen kleine Mengen ab 0,1 Mol freie Base die Ausbeute. Vorzugsweise soll 0,5 bis 6 Mol, sehr bevorzugt 2 bis 3 Mol Base pro Mol Thioharnstoff zugesetzt werden.

Die Konzentration der Reaktionslösung bezüglich Thioharnstoff soll bevorzugt 0,2 bis 6 Mol/l, vorteilhaft 0,5 bis 4 Mol/l, betragen. Das Molverhältnis von Thioharnstoff zu Alkaliformylessigester kann 0,8 bis 1,2 zu 1, vorzugsweise 0,9 bis 1,1 zu 1 sein.

Als Reaktionspunkt fällt das Alkalisalz des 4-Hydroxy-2-mercaptopyrimidins an, das gut wasserlöslich ist. Dieses wird direkt mit einer säßrigen Wasserstoffperoxid-Lösung versetzt. Die Konzentration der Wasserstoffperoxid-Lösung ist nicht kritisch, sie kann z.B. 5 bis 85 Gew.- % Wasserstoffperoxid betragen. Die Menge des Wasserstoffperoxids soll 2 bis 8 Mol, vorzugsweise 2,5 bis 5 Mol Thioharnstoff betragen. Die Umsetzung mit $H_2O_2$ ist exotherm. Zugabe und Reaktion soll bei Temperaturen von 0 bis 150 °C, vorzugsweise von 10 bis 100 °C, erfolgen.

Die Zugabezeit ist abhängig von der Konzentration der $H_2O_2$ -Lösung, der Zugabetemperatur und der Kühlung des Systems und beträgt im allgemeinen etwa 5 Min. bis 2 Std. Eine Nachreaktion ist im allgemeinen nicht erforderlich, sie kann aber bei Temperaturen von 0 bis 150 °C, vorzugsweise von 10 bis 100 °C, in 5 Min. bis 5 Std. erfolgen. Notwendig zur Erzielung befriedigender Ausbeuten ist die Anwesenheit eines Überschusses von Base. Das Molverhältnis Gesamtbase zu eingesetztem Thioharnstoff soll 2,5 bis 8, vorzugsweise 3,5 bis 5 zu 1, betragen. Dabei wird die in Form des Alkaliformylessigsäurealkylesters vorliegende und die bereits zugesetzte Base mitberechnet. Als Base dient vorzugsweise Alkalihydroxid, besonders Natriumhydroxid oder deren wässrige Lösung, gegebenenfalls auch Ammoniumhydroxid, Alkalikarbonate etc. Die Base kann schon vor der Umsetzung von Alkaliformylessigsäurealkylester mit Thioharnstoff zugegeben werden oder nach dieser Umsetzung oder sie kann zusammen mit dem Wasserstoffperoxid zugeführt werden.

Zur Freisetzung des Uracils wird nach der Umsetzung mit Wasserstoffperoxid mit Säuren, vorzugsweise mit Mineralsäuren bzw. deren wäßrigen Lösungen auf einen pH-Wert von weniger als 6, vorzugsweise zwischen 3 und 0,5 eingestellt, wobei das Uracil ausfällt.

Als Mineralsäuren kommen z. B. Schwefelsäure, Salzsäure, Salpetersäure oder Phosphorsäure in Frage. Die Zugabe der Säure kann bei Temperaturen von 0 bis 150 °C, vorzugsweise von 10 bis 100 °C in kurzer Zeit erfolgen. Die Nachreaktionszeit beträgt 0 bis 2 Stunden.

Durch Filtration und Waschen mit Wasser bis zur Salzfreiheit und anschließender Trocknung wird Uracil in hoher Ausbeute mit hoher Reinheit erhalten. Die erzielten Ausbeuten können 80 % und mehr an sehr reiner Substanz erreichen. Das erscheint bemerkenswert im Hinblick auf die mehrstufige Synthese und den einfach zugängigen Ausgangsstoff.

## Beispiel 1

73,0 g (0,5 Mol) Natriumformylessigsäuremethylester (Gehalt 85 Gew.- %) wird portionsweise bei 25 °C zu einer Lösung von 38 g (0,5 Mol) Thioharnstoff und 40 g (1 Mol) Natriumhydroxid in 150 ml Wasser gegeben. Nach 2 Stunden Reaktion bei 25 °C werden 170 g (1,5 Mol) einer 30-Gew. %-igen wäßrigen Wasserstoffperoxid-Lösung in 1/2 Stunde zugegeben, wobei die Temperatur ansteigt und durch Kühlung auf 50 °C gehalten wird. Nach Zugabe des $H_2O_2$ wird auf 20 °C abgekühlt und mit konzentrierter Salzsäure auf den pH-Wert 1 eingestellt. Dabei fällt Uracil als farbloser Feststoff aus, der abfiltriert mit Wasser salzfrei gewaschen und getrocknet wird.

Ausbeute : 44,4 g (79,3 % d. Th.) Schmelzpunkt 335 °C (Zersetzung)

## Beispiel 2

81,2 g (0,5 Mol) Natriumforylessigsäureethylester (Gehalt 85 Gew.- %) wird analog Beispiel 1 umgesetzt und aufgearbeitet.

Ausbeute : 41,7 g (74,5 % d. Th.)

## Beispiel 3

73,0 g (0,5 Mol) Natriumformylessigsäuremethylester (Gehalt 85 Gew.- %) wird wie in Beispiel 1,

jedoch unter Zugabe von 20 g NaOH in 80 ml Wasser zur Reaktion gebracht. Nach einer Stunde Reaktion bei 30 °C werden 40 g NaOH in 50 ml Wasser und 170 g (1,5 Mol) $H_2O_2$ als 30-Gew.%-ige wäßrige Lösung in einer halben Stunde zugegeben, auf 60 °C gehalten und auf 20 °C abgekühlt. Die Zugabe von HCl erfolgt gemäß Beispiel 1.

Ausbeute : 44,9 g (80,1 % d. Th.)

**Patentansprüche**

1. Verfahren zur Herstellung von Uracil der Formel

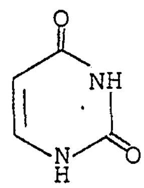

durch Umsetzung von Alkaliformylessigsäurealkylester

MeO—CH=CH—COOR (2)

worin Me ein Alkalimetall, bevorzugt Natrium oder Kalium, und R einen Alkylrest mit 1 bis 8 Kohlenstoffatomen, bevorzugt einen Methyl- oder Ethylrest, bedeuten, mit Thioharnstoff zum Alkalisalz des 2-Mercapto-4-hydroxypyrimidins

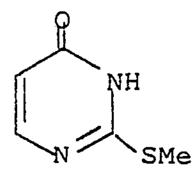

(3)

worin Me dieselbe Bedeutung wie in Formel (2) hat, dadurch gekennzeichnet, daß ohne Isolierung der Stoffe der Formel (3) deren entstandenen Reaktionslösung mit Wasserstoffperoxid in wäßriger Lösung in Gegenwart von mindestens 2 Mol Base zur Reaktion gebracht, anschließend durch Ansäuern auf einen pH-Wert von 6 oder weniger Uracil gebildet und gewonnen wird.

2. Verfahren nach Anspruch 1, dadurch gekennzeichnet, daß das Molverhältnis Wasserstoffperoxid zu Thioharnstoff 2 bis 8, vorzugsweise 2,5 bis 5 zu 1 beträgt.

3. Verfahren nach Anspruch 2, dadurch gekennzeichnet, daß die Reaktion mit Wasserstoffperoxid bei 0 bis 150 °C, vorzugsweise bei 10 bis 100 °C, erfolgt.

4. Verfahren nach Anspruch 2, dadurch gekennzeichnet, daß bei der Reaktion mit Wasserstoffperoxid das Molverhältnis Gesamtbase zu Thioharnstoff 2,5 bis 8 zu 1, vorzugsweise 3,5 bis 5 zu 1, beträgt.

5. Verfahren nach Anspruch 1, dadurch gekennzeichnet, daß das Uracil nach der Umsetzung mit Wasserstoffperoxid durch Ansäuern auf einen pH-Wert von vorzugsweise 3 oder weniger freigesetzt wird.

**Claims**

1. Process for the preparation of uracil of the formula

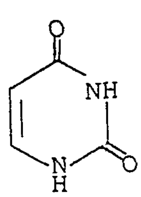

(1)

by reaction of alkali formylacetic acid alkyl esters

MeO—CH=CH—COOR (2)

wherein Me signifies an alkali metal, preferably sodium or potassium, and R signifies an alkyl residue with

1 to 8 carbon atoms, preferably a methyl or ethyl residue, with thiourea to form the alkali salt of 2-mercapto-4-hydroxypyrimidine

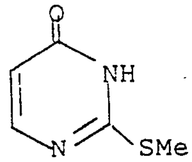

(3)

wherein Me has the same meaning as in formula (2), characterised in that, without isolation of the substances of formula (3), their resulting reaction solution is reacted with hydrogen peroxide in aqueous solution in the presence of at least 2 mol of base, then uracil is formed by acidification to a pH of 6 or less and is recovered.

2. Process according to claim 1, characterised in that the molar ratio of hydrogen peroxide to thiourea amounts to 2 to 8, preferably 2.5 to 5 : 1.

3. Process according to claim 2, characterised in that the reaction with hydrogen peroxide takes place at 0 to 150 °C, preferably at 10 to 100 °C.

4. Process according to claim 2, characterised in that the molar ratio of total base to thiourea in the reaction with hydrogen peroxide amounts to 2.5 to 8 : 1, preferably 3.5 to 5 : 1.

5. Process according to claim 1, characterised in that the uracil is liberated after the reaction with hydrogen peroxide by acidification to a pH value of preferably 3 or less.

**Revendications**

1. Procédé pour la préparation de l'uracile de formule :

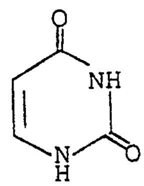

(1)

par réaction d'un ester alkylique d'un acide alcaliformylacétique :

MeO—CH=CH—COOR (2)

où Me représente un métal alcalin, de préférence le sodium ou le potassium, et R représente un reste alkyle ayant 1 à 8 atomes de carbone, de préférence un reste méthyle ou éthyle, avec la thiourée pour obtenir un sel alcalin de la 2-mercapto-4-hydroxypyrimidine :

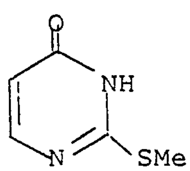

(3)

où Me a le même sens que pour la formule (2), procédé caractérisé en ce que, sans isoler les substances de formule (3), on en fait réagir la solution résultant de cette réaction avec le peroxyde d'hydrogène en solution aqueuse en présence d'au moins 2 moles d'une base, puis, par acidification à un pH égal ou inférieur à 6, on forme et récupère l'uracile.

2. Procédé selon la revendication 1, caractérisé en ce que le rapport molaire du peroxyde d'hydrogène à la thiourée est de 2 à 8, avantageusement de 2,5 à 5 pour 1.

3. Procédé selon la revendication 2, caractérisé en ce que la réaction avec le peroxyde d'hydrogène a lieu à une température de 0 à 150 °C, avantageusement de 10 à 100 °C.

4. Procédé selon la revendication 2, caractérisé en ce que, lors de la réaction avec le peroxyde d'hydrogène, le rapport molaire de la base totale à la thiourée est de 2,5 à 8 pour 1, avantageusement de 3,5 à 5 pour 1.

5. Procédé selon la revendication 1, caractérisé en ce qu'on met en liberté l'uracile, après la réaction avec le peroxyde d'hydrogène, par acidification à un pH qui est de préférence égal ou inférieur à 3.